Europäisches Patentamt

European Patent Office

Office européen des brevets

(11) Publication number: **0 492 172 A1**

# EUROPEAN PATENT APPLICATION

(21) Application number: **91120370.1**

(22) Date of filing: **28.11.91**

(51) Int. Cl.5: **A61B 17/04, A61L 31/00**

(30) Priority: **24.12.90 US 632554**

(43) Date of publication of application:
**01.07.92 Bulletin 92/27**

(84) Designated Contracting States:
**BE CH DE DK ES FR GB GR IT LI NL SE**

(71) Applicant: **AMERICAN CYANAMID COMPANY**
**1937 West Main Street P.O. Box 60**
**Stamford Connecticut 06904-0060(US)**

(72) Inventor: **Corriveau, Paul G.**
**29 Hideaway Lane**
**Sparta, New Jersey 07871(US)**
Inventor: **Riley, John A.**
**21 Mist Hill Drive**
**Brookfield, Connecticut 06804(US)**

(74) Representative: **Wächtershäuser, Günter, Dr.**
**Tal 29**
**W-8000 München 2(DE)**

(54) **Molded or extruded buttressing pledget.**

(57) A buttressing pledget (1) is molded or extruded. In one embodiment, the buttressing pledget (1) has at least one planar surface and an opening (2) that is essentially perpendicular to the at least one planar surface. The pledget (1) can be manufactured from a synthetic elastomeric material, and/or have two openings (2,3) and a protuberance (10,11) adjacent to the proximal ends. The protuberance (10,11) functions as a bumper or reinforcing element.

FIG. 1

This invention relates to a buttressing pledget. The buttressing pledget is manufactured from a synthetic elastomeric material. The elastomeric material can be nonporous and/or a silicone rubber. The pledget can also be radiolucent or radiopaque by the addition of barium sulfate. This invention also relates to a buttressing pledget in combination with a surgical suture or ligature.

The material used to manufacture a surgical pledget has remained relatively constant in the prior art. Probably to avoid irritation to the tissue, the surgical community prefers a soft, loose, fibrous material. A coreless braided and woven pledget are also disclosed in the prior art. Both the braided and the woven pledget are manufactured from a Dacron* (DuPont, DE, USA) fiber.

As the surgical community and wound closure industry sought better solutions to health problems, ne uses of the pledget began to appear. One of these new uses was a buttress during surgery. This new use gave rise to the buttressing pledget as an article of manufacture, which has achieved a separate status in the art. The packaging of a buttressing pledget in combination with a surgical suture is disclosed in the prior art.

The claims in this application are limited to a buttressing pledget. The claimed pledgets can be molded or extruded and have several advantages over the known prior art pledgets. One advantage is that the buttressing pledgets described in this application can be nonfibrous. Because they can be nonfibrous, the problem of exfoliation is essentially eliminated. Another advantage is that the molded or extruded buttressing pledgets can be nonporous. Thus, the problem of tissue ingrowth, which can limit or at least interfere with the wound closure process, is essentially eliminated.

Yet another advantage of a molded or extruded buttressing pledget is that a variety of shapes can be obtained. The availability of various shapes may enhance the efficacy of a known surgical procedure. The shapes described in two dimensions include but are not limited to square, rectangular, round, oval and elliptical, and in three dimensions, the shapes include tubular. Another advantage of a molded or extruded pledget is that is can be manufactured with a more consistent thickness. This advantage can be critical, especially where the pledget is small in size and/or the surgical procedure is major, such as a cardiovascular operation.

Still another advantage is that the buttressing pledget can be manufactured from a radiopaque material. The advantage of a radiopaque material is apparent to the surgical user. For example, in cardiovascular surgery it may be important, if not critical, to know the postoperative location of the pledgets that were used. Knowing the postoperative location, the surgeon or medical technologist can make a prognosis of the patient's wound healing process.

An X-ray photograph, a CAT scan or a similar image can identify the pledget's radiopaque material, and thus show the surgeon or technologist the location of the pledgets. This in turn can indicate that the suturing or stapling technique used by the surgeon is efficacious to the wound healing process.

A further advantage is that the holes formed through a molded or extruded pledget of this invention can be built up around the edges such that the pledget is structurally stronger in these places than the prior art buttressing pledgets.

A natural rubber has been disclosed for use as a latex in a neuropledget. The latex is sprayed onto a surface to form a layer. The neuropledget is manufactured by combining the latex layer with an absorbent fibrous material. Because the utility is not a buttressing pledget, because the natural rubber has to be formed into a latex, because the latex is sprayed onto a layer, because the layer is combined with a fibrous composition, this neuropledget is not material to the inventions claimed in this application. Other prior art compositions that have been used to manufacture buttressing pledgets include a Teflon* (DuPont, DE, USA) or polyurethane fibrous mat.

A pledget comprising a radiopaque material is disclosed in the prior art. The prior art use of this radiopaque pledget is limited to an arterial embolus and specifically the embolization of an arteriovenous malformation. Also disclosed in the prior art are nonpledget medical uses of a silicone rubber.

In summary, the surgical community continues to seek a buttressing pledget which will make surgical procedures more efficacious. To that end and not necessarily in any order of priority, the following embodiments summarize the inventions in this application:

1. An article of manufacture comprising a buttressing pledget manufactured from a synthetic elastomeric material.

2. The article of embodiment 1 wherein the synthetic elastomeric material is nonporous.

3. The article of embodiment 2 wherein the synthetic elastomeric material is radiolucent.

4. The article of embodiment 2 wherein the buttressing pledget is radiopaque.

5. The article of embodiment 1 to 4 wherein the synthetic elastomeric material is a silicone rubber.

6. An article of manufacture comprising a buttressing pledget in combination with a surgical suture or ligature, the buttressing pledget manufactured from a radiolucent, nonporous silicone rubber.

7. The article of embodiment 6 comprising a heat-stable silicone rubber.

8. An article of manufacture comprising a buttressing pledget in combination with a wound closure device, the buttressing pledget manufactured from a nonporous, synthetic elastomer and a radiopaque material.

9. The article of embodiment 8 wherein the wound closure device comprises at least one filament.

10. The article of embodiment 9 wherein the wound closure device comprises a monofilament.

11. The article of embodiment 10 wherein the wound closure device is a surgical suture or ligature.

12. The article of embodiment 9 wherein the wound closure device is a braided suture or ligature.

13. The article of embodiments 1 to 4 and 6 to 12 comprising a molded buttressing pledget.

14. The article of embodiment 13 comprising an injection molded buttressing pledget.

15. The article of embodiment 13 comprising a compression molded buttressing pledget.

16. The article of embodiments 1 to 4 and 6 to 12 comprising an extruded buttressing pledget.

17. The article of embodiment 9 wherein the nonporous, synthetic elastomer is a silicone rubber.

18. The article of embodiment 17 comprising a heat-stable silicone rubber.

19. The article of embodiment 7 or 18 wherein the heat-stable silicone rubber is a mixture comprising 1,1,1-trimethyl-N-(trimethylsilyl)-silanamine, and hydrolysis products of 1,1,1-trimethyl-N-(trimethylsilyl)-silanamine and silica.

20. The article of embodiment 19 wherein the mixture comprises 1,1,1-trimethyl-N-(trimethylsilyl)-silanamine, hydrolysis products of 1,1,1-trimethyl-N-(trimethylsilyl)-silanamine and silica, a methyl substituted siloxane and a methyl substituted silicone.

21. The article of embodiment 19 wherein the mixture comprises 1,1,1-trimethyl-N-(trimethylsilyl)-silanamine, hydrolysis products of 1,1,1-trimethyl-N-(trimethylsilyl)-silanamine and silica, and a dimethyl, methyl hydrogen siloxane copolymer.

22. The article of embodiment 4, 8 to 12, 17 or 18 wherein the radiopaque material is barium sulfate.

23. An article of manufacture comprising a molded or extruded buttressing pledget.

24. The article of embodiment 23 having at least one planar surface.

25. The article of embodiment 24 having at least one opening, the at least one opening being essentially perpendicular to the at least one planar surface.

26. The article of embodiment 23 wherein the molded or extruded buttressing pledget has a lengthwise direction and at least one opening, the at least one opening being essentially parallel to the lengthwise direction.

27. The article of embodiment 25 comprising two planar surfaces and one opening, the opening being essentially perpendicular to the two planar surfaces, said opening having a proximal end and a distal end, and a protuberance adjacent to either or both the proximal and distal end.

28. An article of manufacture comprising a molded or extruded buttressing pledget, the buttressing pledget having a hexahedral form and at least two openings, the at least two openings being essentially parallel to each other.

29. The article of embodiment 28 wherein said buttressing pledget has the form of a rectangular parallelpiped.

30. The article of embodiment 28 wherein said buttressing pledget has one concave surface and the remaining surfaces are planar.

31. The article of embodiment 29 or 30 comprising two openings, each of the two openings having a proximal end and a distal end, and a protuberance adjacent to each of the proximal ends.

32. An article of manufacture comprising in combination a wound closure device and a molded or extruded pledget.

33. The article of embodiment 32 wherein the wound closure device comprises at least one filament.

34. The article of embodiment 33 wherein the wound closure device comprises a monofilament.

35. The article of embodiment 34 wherein the wound closure device is a surgical suture or ligature.

36. The article of embodiment 33 wherein the wound closure device is a braided suture or ligature.

37. The article of embodiment 23 to 30 and 32 to 36 comprising a molded buttressing pledget.

38. The article of embodiment 37 comprising an injection molded buttressing pledget.

39. The article of embodiment 37 comprising a compression molded buttressing pledget.

40. The article of embodiment 23 to 30 and 32 to 36 comprising an extruded buttressing pledget.

41. The article of embodiment 23 to 30 and 32 to 36 wherein the pledget comprises a nonporous material.

42. The article of embodiment 41 wherein the nonporous material is radiolucent.

43. The article of embodiment 41 wherein the pledget comprises a radiopaque material.

44. The article of embodiment 43 wherein the radiopaque material is barium sulfate.

Figures 1 and 5 are perspective views of alternative embodiments of the buttressing pledget of this invention;

Figure 2 is a sectional side view along the plane 2-2 of Figure 1, showing the in-vivo use of the pledget with a surgical suture strand;

Figure 3 is a perspective view of another alternative embodiment of the buttressing pledget, having a curvilinear top;

Figure 4 is a sectional side view along the plane 4-4 of Figure 3; and

Figures 6A and 6B are other alternative embodiments of the buttressing pledget of this invention.

Description

The surgical buttressing pledgets used in this invention are made from injection molded silicone materials. The preparation of this material requires a compounding unit to mix the silicone resins, an injection molding unit, silicon materials, and a mold.

Molds can be in any three dimensional configuration currently adaptable in any molding process. This particular invention recognizes the following configurations which are currently available in teflon felt material, but is not limited only to these shapes: square, rectangular, circular, ovoid and tubular. According to conventional surgical practice, two holes are strategically placed in the pledget for threading of the surgical suture. Pledgets are supplied prethreaded by the manufacturer or can be threaded by the surgeon or nurse prior to use. A tubular shaped pledget is hollow and requires no holes for threading the suture or ligature.

Silicon materials are mixed in the compounding unit under high pressure at a temperature below 10°C. The mix is then injected into the loading part of the molding unit which has been kept at a temperature of approximately 160°C. Vulcanization takes place in the mold within 0.5 to 3.0 minutes, depending on the mold size and configuration. When the vulcanization is complete, the mold is opened and the molded silicon part is manually removed. Deflashing of the silicon part, if necessary, is performed immediately after removal from the mold. For an implantable device such as this, deflashing is performed under magnification. The molding or extruding of a heat-cured silicone rubber, which limitation is disclosed in embodiments 13 to 16 above, is disclosed in the prior art.

How to make a surgical element comprising a radiopaque material as disclosed in embodiments 4 and 8, above, and specifically of barium sulfate as disclosed in embodiment 22, above, is disclosed in the prior art.

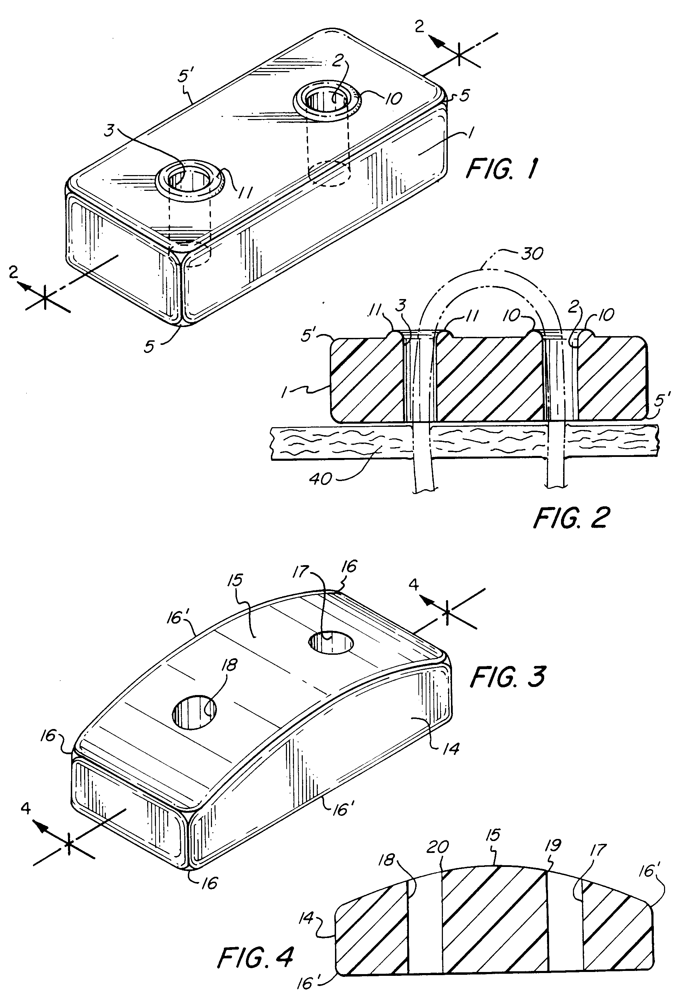

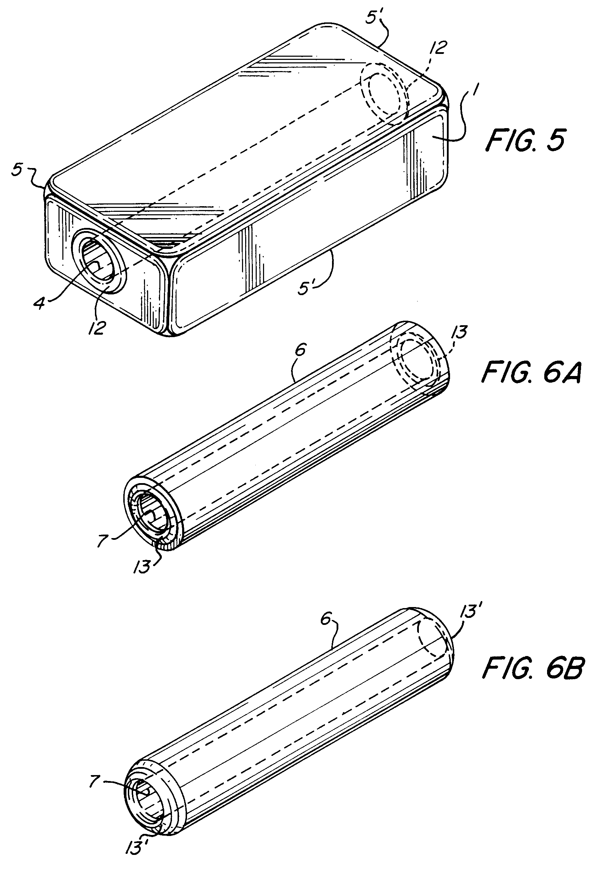

Referring to Figures 1 and 5, the molded or extruded buttressing pledget 1 is rectangular in shape. To facilitate the use of the pledget, Openings 2 and 3 in Figure 1, or 4 in Figure 5 are made through the pledget. As shown in Figures 1 and 2, the two openings 2 and 3 are perpendicular to the top and bottom of the pledget 1. However, it is to be understood that another orientation of the openings, for example a diagonal orientation, is possible.

As shown in Figures 1, 2 and 5, the corners 5 and edges 5' of the molded or extruded buttressing pledget can be rounded. This reduces or even eliminates the risk that during use, the corner(s) or edge(s) will pierce or in some other way traumatize the tissue being approximated.

Referring to Figures 6A and 6B, the molded or extruded buttressing pledget 6 is tubular in shape. The pledget has an opening 7. Preferably, the opening 7 is parallel to the lengthwise direction of the tubular form. However, it is to be understood that another orientation, for example an opening that is perpendicular or diagonal to the lengthwise direction is also within the scope of this invention.

Referring again to Figures 1, 2, and 5, in one embodiment of this invention, a protuberance 10 and 11 at the respective proximal end of each opening 2 and 3 in Figures 1 and 2, and a protuberance 12 at either or both the proximal and distal end of the opening 12 in Figure 5 can be molded directly into the pledget. The term protuberance is intended to be synonymous with the term protrusion. Each of the protuberances (or protrusions) 10 and 11 act as a bumper or reinforcing element. As shown specifically in Figure 2, the protrusions 10 and 11 increase the margin of error in preventing the surgical suture strand 30 from cutting or tearing through the central portion of the pledget 1 formed between the two openings 2 and 3.

The protuberances as described in this application are thus an advance in the buttressing pledget art. There are at least two related reasons for this advance. First, the strength of the pledget is increased and more specifically, the strength is increased at an area of the pledget that the person skilled in the art knows is the most suspect to structural fatigue or failure. Second, the risk of a tear or other fracture of the pledget by a suture strand during the wound healing process is substantially reduced, if not essentially eliminated.

Referring to Figures 6A and 6B, the protrusions shown in Figures 1 and 2 can be adapted to other molded shapes. For example, in Figures 6A and 6B, the protrusions 13 and 13', respectively, can be at one or both ends of the buttressing pledget.

Another embodiment of a molded or extruded buttressing pledget of this invention is shown in Figures 3 and 4. The buttressing pledget 14 has openings 17 and 18. Similar to the rounded corners 5 and edges 5' of Figures 1 and 5, this embodiment can also have rounded corners 16 and edges 16'. The concave top 15 is an alternative means for reinforcing the opening(s) described in Figures 1, 2 and 5 to 6B, above. A description of the concave top 15 as this alternative means is more fully disclosed below.

As shown specifically in Figure 4, the convex top 15 serves the same function as the protrusions 10 and 11 in Figures 1 and 2, or 13 and 13', respectively in Figures 6A and 6B. That is, the raised portions 19 and 20 enable the pledget 14 to be reinforced.

A disclosure of how to use the buttressing pledget of this invention is shown in Figure 2. For conventional use, a surgical suture material 30 is usually prethreaded through the openings 2 and 3 of the pledget 1, as shown. The suture material 30 can be double-armed (that is, have two needles) or single-armed (having a needle at one end). The pledget 1 is then used as a buttress adjacent to the tissue 40, which is being approximated by the single or double-armed surgical suture 30.

Referring again to Figure 2, it is preferred that the protrusions 10 and 11 only be on a side of the pledget 1 that is opposite to the tissue 40. Thus the trauma to the tissue 40 is not increased because the side of the pledget adjacent to the tissue is relatively flat and smooth. However, it is to be understood that the protrusions 10 and 11 could be molded into either end of the openings 2 and 3.

How to use a pledget as a buttress in a surgical procedure is described in the prior art.

A synthetic elastomeric material disclosed in embodiments 1, 2 and 8 above is disclosed in the prior art. Examples of commercial silicone elastomeric compositions include Silplus* (General Electric Co., CT, USA) and Silastic* (Dow Corning Co., Michigan, USA) elastomers.

A molded or extruded buttressing pledget of this invention can be porous. The size of the pores can be controlled by machines and methods known in the prior art. A porous buttressing pledget may be useful in some surgical uses as it probably can provide sites for tissue ingrowth.

The silicone rubber disclosed in embodiments 5, 6 and 17 above, and heat-stable silicone rubber disclosed in embodiments 7 and 18 to 21, is described in the prior art.

Referring to embodiments 19 to 21, above, it is to be understood that each of these mixtures can be generically described as comprising a trimethylated silica. Referring specifically to embodiment 19, the trimethylated silica mixture is described by Chemical Abstracts Service (hereafter "CAS"; American Chemical Soc., Ohio 43210, USA) Registry No. 68909-20-6; embodiments 20 and 21 are described by CAS Registry No. 68037-59-2. That is, the chemical nomenclature described in these embodiments is intended to be synonymous with the respective CAS Registry number. If there is a discrepancy, the nomenclature disclosed in the CAS Registry number takes precedence and is to be used as the description of the embodiment.

## Claims

1. An article of manufacture comprising a molded or extruded buttressing pledget.

2. The article of claim 1 having at least one plannar surface and opening, the at least one opening being essentially perpendicular to the at least one planar surface.

3. The article of claim 1 having a hexahedral form and at least two openings, the at least two openings being essentially parallel to each other.

4. The article of claim 2 having two planar surfaces and one opening, the opening being essentially perpendicular to the two planar surfaces, said opening having a proximal end and a distal end, and a protuberance adjacent to either or both the proximal and distal end, or the article of claim 3 having two openings, each of the two openings having a proximal end and a distal end, and a protuberance adjacent to each of the proximal ends.

5. The article of claim 1 wherein the molded or extruded buttressing pledget comprises a nonporous material.

6. The article of claim 5 wherein the nonporous material is radiolucent, or said molded or extruded buttressing pledget comprises a radiopaque material.

7. The article of claim 1 manufactured from a synthetic elastomeric material.

8. The article of claims 1 to 3 or 5 to 7 in combination with a wound closure device.

9. The article of claim 7 manufactured from a heat-stable silicone rubber in combination with a braided suture or ligature.

10. The article of claim 9 wherein the heat-stable silicone rubber is a mixture comprising 1,1,1-trimethyl-N-(trimethylsilyl)-silanamine, and hydrolysis products of 1,1,1-trimethyl-N-(trimethylsilyl-silanamine and silica; and optionally either a methyl substituted siloxane and a methyl substituted silicone, or a dimethyl, methyl hydrogen siloxane copolymer.

FIG. 1

FIG. 2

FIG. 3

FIG. 4

*FIG. 5*

*FIG. 6A*

*FIG. 6B*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y<br>A | FR-A-2 422 386 (BUSCAYRET)<br>* page 2, line 30 - line 34 *<br>* page 4, line 18 - line 23 *<br>* page 6, line 7 - line 10 *<br>* page 7, line 5; figures * | 1-3,8<br>4,6,9 | A61B17/04<br>A61L31/00 |
| Y<br>A | US-A-4 741 330 (HAYHURST)<br>* column 5, line 4 - line 5 *<br>* column 8, line 1 - line 11 *<br>* column 8, line 39 - line 52; figures * | 1-3,8<br>9 | |
| A | US-A-4 823 794 (PIERCE)<br>* the whole document * | 1-4,8,9 | |
| A | GB-A-2 147 811 (BRISTOL-MYERS)<br>* page 2, line 28 - line 30 *<br>* page 2, line 128 - line 130; claim 3; figures 1,2 * | 7,9 | |
| A | WORLD PATENTS INDEX LATEST<br>Section Ch, Week 8551,<br>Derwent Publications Ltd., London, GB;<br>Class A, AN 85-319398<br>& JP-A-60 221 430 (SHINETSU) 6 November 1985<br>* abstract * | 1,7,9,10 | TECHNICAL FIELDS SEARCHED (Int. Cl.5 )<br><br>A61B<br>A61L |
| A | GB-A-2 063 675 (JURO WADA) | | |

The present search report has been drawn up for all claims

| Place of search | Date of completion of the search | Examiner |
|---|---|---|
| THE HAGUE | 29 JANUARY 1992 | KLEIN C. |

EPO FORM 1503 03.82 (P0401)